(19) Europäisches Patentamt
European Patent Office
Office européen des brevets

(11) EP 0 889 050 A2

(12) EUROPÄISCHE PATENTANMELDUNG

(43) Veröffentlichungstag:
**07.01.1999 Patentblatt 1999/01**

(51) Int. Cl.[6]: **C07F 13/00**, C07F 15/06, C11D 3/39

(21) Anmeldenummer: **98111050.5**

(22) Anmeldetag: **17.06.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **01.07.1997 DE 19728021**

(71) Anmelder: **Clariant GmbH**
**65929 Frankfurt am Main (DE)**

(72) Erfinder: **Nestler, Bernd, Dr.**
**65929 Frankfurt (DE)**

(54) **Metall-Komplexe als Bleichaktivatoren**

(57) Metall-Komplexe der allgemeinen Formel 1

$$[L_nM_mX_p]^zY_q \quad (1)$$

wobei

M für Mangan in der Oxidationsstufe II, III, IV, V und/oder VI oder Kobalt in der Oxidationsstufe II und/oder III oder Eisen in der Oxidationsstufe II und/oder III,
X eine Koordinations- oder Brückengruppe,
Y ein Gegenion in der entsprechenden stöchiometrischen Menge zum Ausgleich einer vorhandenen Ladung z, wobei
z als Ladung des Metall-Komplexes positiv, null oder negativ sein kann,
n und m unabhängig voneinander ganze Zahlen von 1 bis 4,
p eine ganze Zahl von 0 bis 15,
q z/Ladung von Y
L einen Liganden der Formel (2), (3) oder (4)

EP 0 889 050 A2

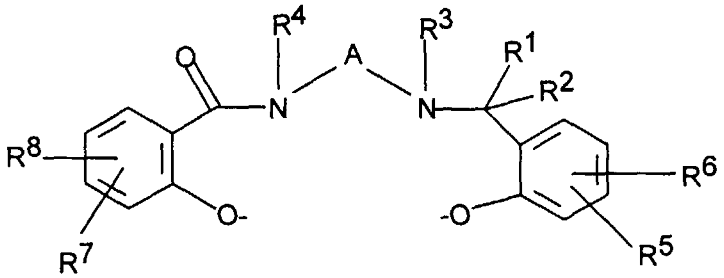

(2)

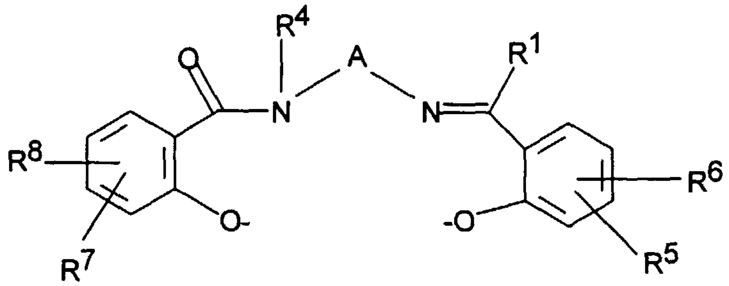

(3)

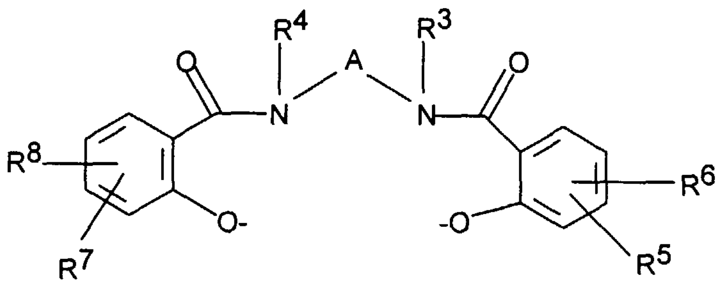

(4)

bedeuten und A und $R^1$ bis $R^8$ die in der Beschreibung genannten Bedeutungen haben.

## Beschreibung

Es ist bekannt, daß sich das Bleichvermögen peroxidischer Bleichmittel in Wasch- und Reinigungsmitteln, wie Wasserstoffperoxid, Perboraten, Percarbonaten, Persilikaten und Perphosphaten und somit die Effizienz dieser Bleichmittel zur Entfernung von Tee-, Kaffee-, Obst- oder Rotweinflecken erst bei höheren Temperaturen von deutlich über 60 °C voll entfaltet. Zur Verbesserung der bei niedrigeren Temperaturen, vor allem unter 60 °C, stark herabgesetzten Bleichwirkung können Verbindungen zur Aktivierung der Peroxidbleichmittel eingesetzt werden. Für diesen Zweck wurden eine Reihe von Übergangsmetallsalzen bzw. entsprechende Komplexe mit meist chelatisierenden Verbindungen vorgeschlagen, doch ist die Wirksamkeit eines Metalls bzw. einer speziellen Kombination von Übergangsmetall und Komplexligand nicht voraussagbar.

Solche Metall-Komplexe zur Aktivierung von Peroxyverbindungen sind beschrieben in US 5314635, US 5244594, US 5114611, US 5114606, US 4728455, EP 717103, EP 6300964, EP 549272, EP 544519, EP 544490, EP 544440, EP 509787, EP 458398, EP 408131, WO 9707191, WO 977192, WO 9615136, WO 9530681.

Gegenstand der vorliegenden Erfindung sind neue Verbindungen der allgemeinen Formel 1

$$[L_nM_mX_p]^zY_q \qquad (1)$$

wobei

- M für Mangan in der Oxidationsstufe II, III, IV, V und/oder VI oder Kobalt in der Oxidationsstufe II und/oder III oder Eisen in der Oxidationsstufe II und/oder III,
- X eine Koordinations- oder Brückengruppe,
- Y ein Gegenion in der entsprechenden stöchiometrischen Menge zum Ausgleich einer vorhandenen Ladung z, wobei
- z als Ladung des Metall-Komplexes positiv, null oder negativ sein kann,
- n und m unabhängig voneinander ganze Zahlen von 1 bis 4,
- p eine ganze Zahl von 0 bis 15,
- q z/Ladung von Y
- L einen Liganden der Formel (2), (3) oder (4)

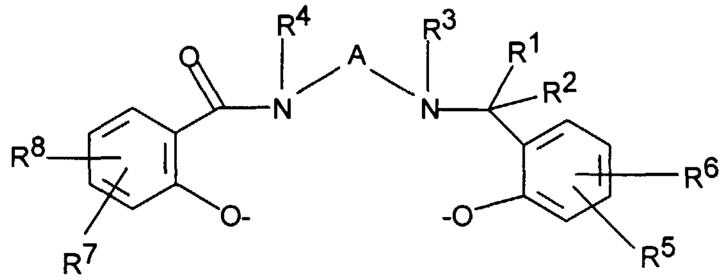

(2)

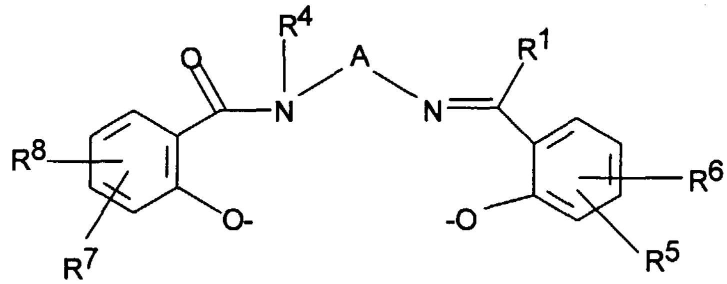

(3)

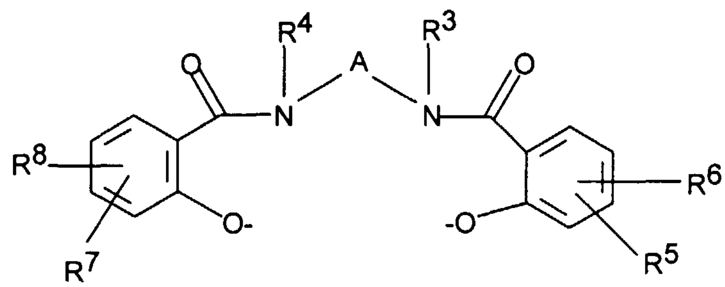

(4)

bedeutet, wobei

$R^1$, $R^2$, $R^3$, $R^4$ unabhängig voneinander Wasserstoff, einen $C_1$- bis $C_{10}$-Alkyl-, Cycloalkyl- oder Arylrest,

$R^5$, $R^6$, $R^7$, $R^8$ unabhängig voneinander Wasserstoff, $C_1$- bis $C_{30}$-Alkyl-, Cycloalkyl- oder Arylreste, $C_1$- bis $C_4$-Alkoxygruppen, substituierte oder unsubstituierte Amino- oder Ammoniumgruppen, Halogenatome, Sulfogruppen, Carboxylgruppen, oder Gruppierungen der Formel $-(CH_2)_r-COOH$, $-(CH_2)_r-SO_3H$, $-(CH_2)_r-PO_3H_2$, $-(CH_2)_l-OH$ bedeuten, wobei r eine ganze Zahl von 0 bis 4 und l eine ganze Zahl von 1 bis 4 bedeutet und die genannten Säuregruppen auch in Salzform vorliegen können und

A für einen $C_2$- bis $C_4$-Alkylenrest, einen $C_5$- bis $C_{10}$-Cycloalkylen- oder einen Arylenrest steht,

ausgenommen Verbindungen der Formel 1 wenn L ein Ligand der Formel 4, M = Co, Fe, A = Ethylen, $R^3$ und $R^4$ Wasserstoff und $R^5$ bis $R^8$ Chlor sind.

X ist vorzugsweise eine der folgenden Gruppen:
$F^-$, $Cl^-$, $Br^-$, $SCN^-$, $OH^-$, $O_2^{2-}$, $O^{2-}$, $O_2^-$, $HOO^-$, $R^9OO^-$, $H_2O$, $SH^-$, $CN^-$, $OCN^-$, $S^{2-}$, $N_3^-$, $NH_3$, $NR^9_3$, $NR^9_2^-$, $R^9O^-$, $R^9COO^-$, $R^9SO_3^-$ und $R^9SO_4^-$, wobei $R^9$ jeweils für Wasserstoff, $C_1$- bis $C_8$-Alkyl, Cycloalkyl oder $C_6$- bis $C_{18}$-Aryl steht. Das Gegenion Y ist vorzugsweise ein Ion der folgenden Formeln:

- bei positivem z: $F^-$, $Cl^-$, $Br^-$ $NO_3^-$, $ClO_4^-$, $SCN^-$, $PF_6^-$, $R^9SO_4^-$, $R^9COO^-$, $R^9SO_3^-$, $BF_4^-$, $BPh_4^-$, $SO_4^{2-}$ und $SO_4^{2-}$;
- bei negativem z: $Li^+$, $Na^+$, $K^+$, $Mg^{2+}$, $Ca^{2+}$, $Al^{3+}$, $NH_4^+$, $R^9NH_3^+$, $R^9{}_2NH_2^+$, $R^9{}_3NH^+$ und $R^9{}_4N^+$,

wobei $R^9$ die zuvor genannte Bedeutung hat.

| | |
|---|---|
| M | ist vorzugsweise Mangan, |
| $R^1$, $R^2$, $R^3$, $R^4$, $R^6$, $R^7$ und $R^8$ | sind bevorzugt Wasserstoff, |
| $R^5$ | ist bevorzugt $C_1$- bis $C_4$-Alkyl-, $C_1$- bis $C_4$-Alkoxy oder eine substituierte oder unsubstituierte Amino- oder Ammoniumgruppe, insbesondere eine Di-$C_1$- bis $C_4$-Alkylaminogruppe und |
| A | ist bevorzugt Ethylen. |

Die Liganden der Formel 4 sind durch Umsetzung von Salicylsäurederivaten wie z.B. Methylester mit Diaminoalkanen zugänglich; Umsetzungen mit N-(2-Hydroxybenzyl)diaminoalkanen ergeben Liganden der Formel 2, analoge Reaktionen sind in Y. A. Ibrahim, A. H. M. Elwahy, Synthesis 503-508 (1993) beschrieben. Eine alternative Herstellung von Liganden der Formel 2 ist die Reduktion von Liganden der Formel 3, die durch Umsetzung von N-(2-Hydroxybenzoyl)diaminoalkanen mit entsprechend substituierten Salicylaldehyden synthetisierbar sind, in Anlehnung an die Angaben in A. Böttcher et al., Z. Naturforsch. 49b, 1089-1100 (1994). Die Herstellung der erfindungsgemäßen Metallkomplexe durch Umsetzung von Mn-, Co- oder Fe-Salzen mit diesen Liganden erfolgt ebenfalls analog zu den Angaben dieser Literaturstelle.

Die erfindungsgemäßen ein- oder mehrkernigen Komplexe der allgemeinen Formel 1 eignen sich hervorragend als Bleich- und Oxidationskatalysatoren, insbesondere in Wasch- und Reinigungsmitteln und bei der Textil- und Papierbleiche. Besonders hervorzuheben sind hier Textilwaschmittel in Form von Pulverwaschmitteln oder als flüssige Formulierungen und Geschirreinigungsmittel. Ein Vorteil der erfindungsgemäßen Bleichkatalysatoren ist hierbei ihre Stabilität gegen Hydrolyse und Oxidation sowie ihre katalytische Wirkung bereits bei niedrigen Temperaturen. Sie verbessern in solchen Formulierungen nicht nur die Bleichwirkung von Wasserstoffperoxid, sondern auch von organischen und anorganischen Peroxy-Verbindungen.

Gegenstand der vorliegenden Erfindung ist demgemäß auch ein Verfahren zum Blechen von verschmutzten Substraten, wobei man das verschmutzte Substrat in wäßriger Bleichflotte mit Peroxyverbindungen und einer wirksamen Menge eines oder mehrerer der erfindungsgemäßen Metall-Komplexe als Bleichkatalysatoren in Kontakt bringt.

Dabei enthält die wäßrige Bleichflotte vorzugsweise diese Metall-Komplexe bezogen, auf das Gewicht der Bleichflotte, in einer Menge von 0,001 bis 100 ppm Metall, insbesondere 0,01 bis 50 ppm Metall, vor allem 0,03 bis 20 ppm Metall (ppm bedeutet parts per million, bezogen auf das Gewicht). Höhere Gehalte an Metallkomplexen, etwa bis zu 500 ppm, können bei industriellen Bleichprozessen, beispielsweise auf dem Textil- oder Papiersektor, zweckmäßig sein. Die zuerst genannten niedrigen Metall-Gehalte beziehen sich vornehmlich auf Haushaltstextilwaschmittel.

Gegenstand der Erfindung ist auch die Verwendung dieser Bleichkatalysatoren in bleichenden Wasch- und Reinigungsmitteln. Diese Wasch- und Reinigungsmittel enthalten neben einer Peroxidverbindung oder einer peroxidfreisetzenden Verbindung und dem Bleichkatalysator üblicherweise auch oberflächenaktive Verbindungen und weitere bekannte Inhaltsstoffe.

Geeignete Peroxide bzw. peroxidfreisetzende Verbindungen sind Alkalimetallperoxide, organische Peroxide wie Harnstoff-Wasserstoffperoxid-Addukte, und anorganische Persalze, wie die Alkaliperborate, -percarbonate, -perphosphate, -persilikate und -persulfate. Besonders bevorzugt sind Natriumperborat-Tetrahydrat und insbesondere Natriumperborat-Monohydrat. Natriumperborat-Monohydrat ist wegen seiner guten Lagerbeständigkeit und seiner guten Löslichkeit in Wasser bevorzugt. Natriumpercarbonat kann aus Umweltschutzgründen bevorzugt sein. Alkylhydroperoxide sind eine weitere geeignete Gruppe von Peroxidverbindungen. Beispiele für diese Stoffe sind Cumolhydroperoxid und t-Butylhydroperoxid. Auch aliphatische oder aromatische Mono- oder Dipercarbonsäuren sowie die entsprechenden Salze eignen sich als Peroxyverbindungen. Beispiele hierfür sind Peroxy-$\alpha$-naphthoesäure, Peroxylaurinsäure, Peroxystearinsäure, N,N-Phthaloylaminoperoxycapronsäure, 1,12-Diperoxydodecandisäure, 1,9-Diperoxyazelainsäure, Diperoxysebacinsäure, Diperoxyisophthalsäure, 2-Decyldiperoxybutan-1,4-disäure und 4,4'-Sulfonylbisperoxybenzoesäure. Weiterhin eignen sich als Peroxyverbindungen anorganische Peroxysäure-Salze, z. B. Kaliummonopersulfat. Mischungen aus zwei oder mehreren dieser Verbindungen sind ebenfalls geeignet.

Die erfindungsgemäßen Wasch- und Reinigungsmittel-Formulierungen enthalten üblicherweise 1 bis 30 Gew.-%, insbesondere 2 bis 25 Gew.-% an Peroxyverbindungen.

Neben den Peroxyverbindungen können die Wasch- und Reinigungsmittel zusätzlich auch sogenannte Bleichaktivatoren in üblichen Mengen (ca. 1 bis 10 Gew.-%) enthalten.

Beispiele für solche Bleichaktivatoren sind Verbindungen mit quaternären Ammonium-Strukturen wie beispielsweise 2-(N,N,N-Triethylammonium)ethyl-4-sulfophenylcarbonat, N-Octyl-N,N-dimethyl-N-10-carbophenoxydecylammoniumchlorid, 3-(N,N,N-Trimethylammonium)-propyl-Natrium-4-sulfophenylcarboxylat und N,N,N-Trimethylammonium-toluyl-oxybenzolsulfonat.

Neben den oben genannten quaternären Ammoniumsalzen sind Ester wie z. B. Acylphenolsulfonate und Acylalkylphenolsulfonate sowie Acylamide als Bleichaktivator bevorzugt. Von besoderem Interesse sind hierbei die in der Praxis gerne eingesetzten Verbindungen Natrium-4-benzoyloxybenzol-sulfonat, N,N,N',N'-Tetraacetylethylendiamin (TAED), Natrium-1-methyl-2-benzoyloxy-benzol-4-sulfonat, Natrium-4-methyl-3-benzoyloxybenzoat, Natriumnonanoyloxybenzol-sulfonat, Natrium-3,5,5-trimethylhexanoyloxybenzo-sulfonat, Benzoylcaprolactam, 2-Phenyl-benz-(4H)1,3-oxazin-4-on, Glucosepentaacetat und Tetraacetylxylose aber auch Ketone sowie nitrilische Aktivatoren.

Als wirksame Menge der Metall-Komplexe der Formel 1 sind üblicherweise Mengen von 0,0001 bis 0,5 Gew.-% Metall, insbesondere 0,00025 bis 0,25 Gew.-% Metall, vor allem 0,0005 bis 0,1 Gew.-% Metall, bezogen auf das Gewicht der Formulierungen, in diesen Wasch- und Reinigungsmittelformulierungen enthalten.

Diese Mengen können je nach landesüblichen Gepflogenheiten leicht schwanken.

Die oberflächenaktive Substanz in den Wasch- und Reinigungsmitteln kann von Naturprodukten abgeleitet sein, wie etwa Seife, oder ist eine synthetische Verbindung aus der Gruppe der anionischen, nichtionischen, amphoteren, (zwitterionischen) oder kationischen oberflächenaktiven Substanzen, oder Mischungen aus diesen. Viele geeignete Substanzen sind kommerziell erhältlich, und sind in der Literatur beschrieben, beispielsweise in "Surface active agents and detergents", Vol. 1 und 2, von Schwartz, Perry und Berch. Der Gesamtanteil der oberflächenaktiven Verbindungen kann bis zu 50 Gew.-% betragen, vorzugsweise 1 Gew.-% bis 40 Gew.-%, insbesondere 4 Gew.-% bis 25 Gew.-% des gesamten Wasch- oder Reinigungsmittels betragen.

Synthetische anionische oberflächaktive Substanzen sind üblicherweise wasserlösliche Alkalimetallsalze von organischen Sulfaten und Sulfonaten mit Alkylresten von etwa 8 bis 22 Kohlenstoffatomen, wobei der Ausdruck "Alkyl" die Alkylsubstituenten von Arylresten einschließt.

Beispiele geeigneter anionischer Detergentien sind Natrium- und Ammoniumalkylsulfonate, speziell die durch Sulfatierung höherer ($C_8$ bis $C_{18}$) Alkohole erhaltenen Sulfate; Natrium- und Ammoniumalkylbenzolsulfonate mit einem Alkylrest von $C_9$ bis $C_{20}$, insbesondere lineare sekundäre Natriumalkylbenzolsulfonate mit einem Alkylrest von $C_{10}$ bis $C_{15}$; Natriumalkylglycerinethersulfate, besonders die Ester der höheren, von Talg- und Kokosnußöl abgeleiteten Alkohole; die Natriumsulfate und -sulfonate der Kokosfettsäuremonoglyceride; Natrium- und Ammoniumsalze der Schwefelsäureester höherer ($C_9$ bis $C_{18}$) oxalkylierter, insbesondere der mit Ethylenoxid oxalkylierten Fettalkohole; die Reaktionsprodukte der Veresterung von Fettsäuren mit Isethionsäure und nachfolgender Neutralisierung mit Natriumhydroxid; Natrium- und Ammoniumsalze der Fettsäureamide des Methyltaurins; Alkan-Monosulfonate wie diejenigen aus der Reaktion von $\alpha$-Olefinen ($C_8$-$C_{20}$) mit Natriumbisulfit und diejenigen aus der Reaktion von Paraffinen mit $SO_2$ und $Cl_2$ mit anschließender basischer Hydrolyse, wobei ein Gemisch verschiedener Sulfonate entsteht; Natrium- und Ammoniumdialkylsulfosuccinate mit Alkylresten von $C_7$ bis $C_{12}$; und Olefinsulfonate, die bei der Reaktion von Olefinen, insbesondere $C_{10}$- bis $C_{20}$-$\alpha$-Olefinen, mit $SO_3$ und nachfolgender Hydrolyse der Reaktionsprodukte entstehen. Die bevorzugten anionischen Detergentien sind Natriumalkylbenzolsulfonate mit Alkylresten von $C_{15}$ bis $C_{18}$, und Natriumalkylethersulfate mit Alkylresten von $C_8$ bis $C_{18}$.

Beispiele für geeignete nichtionische oberflächenaktive Verbindungen, die bevorzugt zusammen mit anionischen obenflächenaktiven Verbindungen benutzt werden, sind insbesondere die Reaktionsprodukte von Alkylenoxiden (gewöhnlich Ethylenoxid) mit Alkylphenolen (Alkylreste von $C_5$ bis $C_{22}$), wobei die Reaktionsprodukte im allgemeinen 5 bis 25 Ethylenoxid (EO)-Einheiten im Molekül enthalten; die Reaktionsprodukte aliphatischer ($C_8$ bis $C_{18}$) primärer oder sekundärer, linearer oder verzweigter Alkohole mit Ethylenoxid, mit im allgemeinen 6 bis 30 EO, und die Additionsprodukte von Ethylenoxid an Reaktionsprodukte aus Propylenoxid und Ethylendiamin. Andere nichtionische oberflächenaktive Verbindungen sind Alkylpolyglycoside, langkettige tertiäre Aminoxide, langkettige tertiäre Phosphinoxide und Dialkylsulfoxide.

Amphotere oder zwitterionische oberflächenaktive Verbindungen können ebenfalls in den erfindungsgemäßen Zusammensetzungen verwendet werden, was aber wegen ihrer hohen Kosten meistens nicht erwünscht ist. Wenn amphotere bzw. zwitterionische Verbindungen verwendet werden, so geschieht das in der Regel in kleinen Mengen in Zusammensetzungen, die hauptsächlich anionische und nichtionische Tenside enthalten.

Auch Seiten können in den erfindungsgemäßen Zusammensetzungen verwendet werden, vorzugsweise mit einem Anteil von weniger als 25 Gew.-%. Sie sind besonders geeignet in geringen Mengen in binären (Seife/nichtionisches Tensid) oder in ternären Mischungen zusammen mit nichtionischen oder gemischten synthetischen anionischen und nichtionischen Tensiden. Die verwendeten Seifen sind bevorzugt die Natriumsalze, und weniger bevorzugt die Kaliumsalze gesättigter und ungesättigter $C_{10}$- bis $C_{24}$-Fettsäuren, oder deren Mischungen. Die Anteile solcher Seifen können von 0,5 Gew.-% bis 25 Gew.-% betragen, geringere Mengen von 0,5 Gew.-% bis 5 Gew.-% sind im allgemeinen ausreichend zur Schaumkontrolle. Seifenanteile zwischen etwa 2 % und etwa 20 %, besonders zwischen etwa 5 % und etwa 10 %, haben einen positiven Effekt. Dieses ist besonders der Fall in hartem Wasser, wo die Seife als zusätzliche Buildersubstanz dient.

Die Wasch- und Reinigungsmittel enthalten im allgemeinen auch einen Builder. Als Builder kommen in Betracht: Calcium-bindende Stoffe, Fällungsmittel, Calcium-spezifische Ionenaustauscher und deren Mischungen. Beispiele für Calciumbindende Stoffe umfassen Alkalimetallpolyphosphate, wie Natriumtripolyphosphat; Nitrilotriessigsäure und ihre wasserlöslichen Salze; die Alkalimetallsalze der Carboxymethyloxybernsteinsäure, Ethylendiamintetraessigsäure, Oxydibernsteinsäure, Mellithsäure, Benzopolycarbonsäuren, Zitronensäure; und Polyacetalcarboxylate, wie in U. S. Pat. 4144226 und 4146495 offenbart. Beispiele für Fällungsmittel sind Natriumorthophosphat, Natriumcarbonat und Seifen aus langkettigen Fettsäuren.

Beispiele für Ionenaustauscher, die für Calcium spezifisch sind, sind die verschiedenen Arten von wasserunlöslichen, kristallinen oder amorphen Aluminiumsilicate, von denen die Zeolithe die bekanntesten Vertreter sind.

Die Buildersubstanzen können von 5 Gew.-% bis 80 Gew.-% vorhanden sein, bevorzugt ist ein Anteil von 10 Gew.-% bis 60 Gew.-%.

Neben den bereits erwähnten Inhaltsstoffen können die Wasch- und Reinigungsmittel konventionelle Zusatzstoffe in Mengen enthalten, die man üblicherweise in solchen Mitteln vorfindet. Beispiele hierfür sind Schaumbildner, wie etwa Alkanolamide, besonders die Monoethanolamide aus Palmkernöl-Fettsäuren und Kokosnuß-Fettsäuren; schaumverhindernde Substanzen, wie etwa Alkylphosphate und -silicone; Vergrauungsinhibitoren und ähnliche Hilfsmittel, wie etwa Natriumcarboxymethylcellulose und Alkyl- oder substituierte Alkylcelluloseether; Stabilisatoren, wie Ethylendiamintetraessigsäure; Weichmacher für Textilien; anorganische Salze, wie Natriumsulfat; und, in üblicherweise kleinen Mengen, fluoreszierende Stoffe, Parfüme, Enzyme wie Proteasen, Cellulasen, Lipasen und Amylasen, Desinfektionsmittel und Farbstoffe. Die Bleichkatalysatoren dieser Erfindung können in einer Vielzahl von Produkten eingesetzt werden. Diese umfassen Textilwaschmittel, Textilbleichmittel, Oberflächenreiniger, Toilettenreiniger, Geschirrspülmaschinenreiniger, und auch Gebißreiniger. Die Waschmittel können in fester Form oder flüssiger Form vorliegen.

Es ist aus Gründen der Stabilität und Handhabbarkeit vorteilhaft, die Bleichaktivatoren in Form von Granulaten zu verwenden, die neben dem Bleichkatalysator ein Bindemittel enthalten. Verschiedene Methoden, solche Granulate herzustellen, sind in der Patentliteratur beschrieben, so beispielsweise in Kanada Pat. Nr. 1102966, GB 1561333, US 4087369, EP 240057, EP 241962, EP 101634 und EP 62523.

Die die erfindungsgemäßen Bleichkatalysatoren enthaltenden Granulate werden im allgemeinen der Waschmittelzusammensetzung zusammen mit den anderen, trockenen Bestandteilen wie etwa Enzymen, anorganischen Peroxidbleichmitteln zugesetzt. Die Waschmittelzusammensetzung, zu der die Katalysatorgranulate zugegeben werden, kann auf verschiedenen Wegen erhalten werden, wie etwa Mischen der trockenen Komponenten, Extrudieren, Sprühtrocknung.

In einer weiteren Ausführungsform sind die erfindungsgemäßen Bleichkatalysatoren besonders geeignet für nichtwäßrige flüssige Waschmittel, zusammen mit einer bleichenden Peroxidverbindung, etwa Natriumperborat, um dem Waschmittel ein großes Reinigungsvermögen für Gewebe und Textilien zu verleihen. Derartige nichtwäßrige, flüssige Waschmittel, die pastöse und gelatinöse Detergentienzusammensetzungen mit einschließen, sind beispielsweise in US 2864770, US 2940938, US 4772412, US 3368977, GB 1205711, GB 1370377, GB 1270040, GB 1292352, GB 2194536, DE 2233771, EP 28849 beschrieben. Es handelt sich dabei um Zusammensetzungen in Form eines nichtwäßrigen, flüssigen Mediums, in dem eine feste Phase dispergiert sein kann. Das nicht wäßrige, flüssige Medium kann eine flüssige, oberflächenaktive Substanz sein, vorzugsweise eine nichtionische oberflächenaktive Substanz, ein nicht polares flüssiges Medium wie etwa flüssiges Paraffin, ein polares Lösungsmittel, wie etwa Polyole, zum Beispiel Glycerin, Sorbitol, Ethylenglycol, eventuell in Verbindung mit niedermolekularen einwertigen Alkoholen wie Ethanol oder Isopropanol oder Mischungen daraus.

Die feste Phase kann aus Buildersubstanzen, Alkalien, abrasiven Stoffen, Polymeren und festen ionischen oberflächenaktiven Verbindungen, Bleichmitteln, fluoreszierenden Stoffen und anderen üblichen festen Inhaltsstoffen bestehen.

Die folgenden Beispiele sollen einen Überblick über die Ausführungsformen der Erfindung geben.

Beispiel 1: N-(2-Hydroxybenzoyl)-N'-(2-hydroxybenzyliden)-1,2-diaminoethan-Mangan-Komplex

Eine Lösung von 21,6 g N-(2-Hydroxybenzoyl)-1,2-diaminoethan (Hydrochlorid), (hergestellt in Anlehnung an: B. Gutkowska, S. Biniecki, Acta Polon. Pharm. 19, 243-249 (1962) [Chem. Abstr. 59, 7425 (1963)]) 12,2 g Salicylaldehyd und 11,0 g Triethylamin in 100 ml Toluol wurde drei Stunden unter Rückfluß zum Sieden erhitzt. Das Solvens wurde im Vakuum entfernt, der verbliebene Rückstand mit Wasser chloridfrei gewaschen und im Vakuum getrocknet. Ausbeute: 28,0 g N-(2-Hydroxybenzoyl)-N'-(2-hydroxybenzyliden)-1,2-diaminoethan.

Von dieser Verbindung wurden 1,14 g in 50 ml Ethanol gelöst und portionsweise 0,98 g Mangen-(II)-acetat (Tetrahydrat) zugegeben. Nach einer Stunde Erhitzen unter Rückfluß und achtstündigem Stehenlassen bei Raumtemperatur wurde das Solvens am Rotationsverdampfer entfernt. Das Produkt wurde aus dem Rückstand mit Methylenchlorid extrahiert und durch Ausrühren mit Isopropanol gereinigt. Erhalten wurden 1,0 g N-(2-Hydroxybenzoyl)-N'-(2-hydroxybenzyliden)-1,2-diaminoethan-Mangan-Komplex in Form eines dunkel-braunen amorphen Feststoffs.

Beispiel 2: N-(2-Hydroxybenzoyl)-N'-(2-hydroxybenzyliden)-1,2-diaminoethan-Mangan-Komplex

Der N-(2-Hydroxybenzoyl)-N'-(2-hydroxybenzyliden)-1,2-diaminoethan-Mangan-Komplex wurde wie in Beispiel 1 beschrieben synthetisiert; dabei wurde in der Umsetzung Mangan-(III)-acetat (Dihydrat) anstelle von Mangan-(II)-acetat (Tetrahydrat) eingesetzt.

Beispiel 3: N-(2-Hydroxybenzoyl)-N'-(2-hydroxybenzyl)-1,2-diaminoethan-Mangan-Komplex

Zu einer Lösung von 8,31 g N-(2-Hydroxybenzyl)-1,2-diaminoethan [hergestellt in Anlehnung an: A. Böttcher et al., Z. Naturforsch. 49b, 1089-1100 (1994)] in 50 ml Toluol wurden 11,4 g Salicylsäuremethylester zugetropft und das erhaltene Gemisch 45 Minuten unter Rückfluß zum Sieden erhitzt. Beim Abkühlen fiel ein Feststoff aus; dieser wurde abgetrennt, mit Ethanol/Wasser gewaschen und getrocknet. Man erhielt 3,59 g N-(2-Hydroxybenzoyl)-N'-(2-hydroxybenzyl)-1,2-diaminoethan. 1,72 g der so erhaltenen Verbindung wurden in einem Gemisch aus 30 ml Ethanol und 7 ml Dimethylformamid gelöst und portionsweise mit 1,49 g Mangan-(II)-acetat (Tetrahydrat) versetzt. Mach dreistündigem Rühren in der Siedehitze wurde filtriert und das Solvens am Rotationsverdampfer (Wasserstrahlvakuum) entfernt. Erhalten wurden 1,66 g N-(2-Hydroxybenzoyl)-N'-(2-hydroxybenzyl)-1,2-diaminoethan-Mangan-Komplex in Form eines dunkelbraunen amorphen Feststoffs.

Beispiel 4: N-(2-Hydroxybenzoyl)-N'-(2-hydroxybenzyl)-1,2-diaminoethan-Mangan-Komplex

Der N-(2-Hydroxybenzoyl)-N'-(2-hydroxybenzyl)-1,2-diaminoethan-Mangan-Komplex wurde wie in Beispiel 3 beschrieben synthetisiert; anstelle von Mangan-(II)-acetat (Tetrahydrat) wurde dabei in der Umsetzung Mangan-(III)-acetat (Dihydrat) eingesetzt.

Beispiel 5: N-(2-Hydroxybenzoyl)-N'-(3,5-ditert.butyl-2-hydroxybenzyl)-1,2-diaminoethan-Mangan-Komplex

Eine Suspension von 3,96 g N-(2-Hydroxybenzoyl)-N'-(3,5-ditert.butyl-2-hydroxybenzyliden)-1,2-diaminoethan in 25 ml Ethanol wurde mit 2 Tropfen wäßriger Natronlauge versetzt und bei einer Temperatur von 5 - 10°C portionsweise 400 mg Natriumborhydrid zugegeben. Nach 14 h Stehenlassen bei Raumtemperatur wurde das Solvens im Vakuum entfernt. Der erhaltene Rückstand wurde mit 50 ml Wasser aufgeschlämmt, mit verdünnter Salzsäure neutralisiert, abgesaugt und getrocknet. Erhalten wurden 3,80 g N-(2-Hydroxybenzoyl)-N'-(3,5-ditert.butyl-2-hydroxybenzyl)-1,2-diamino-ethan.
1,59 g dieser Verbindung wurden in 40 ml Ethanol gelöst und portionsweise mit 980 mg Mangan-(II)-acetat (Tetrahydrat) versetzt. Nach einer Stunde Refluxieren und vierzehnstündigem Stehenlassen bei Raumtemperatur wurde das Solvens evaporiert. Das so erhaltene Rohprodukt wurde in 1,2-Dichlorethan gelöst, die unlöslichen Bestandteile abgetrennt, das Lösungsmittel im Vakuum entfernt und der Rückstand mit Petrolether gewaschen. Man erhielt 1,50 g N-(2-Hydroxybenzoyl)-N'-(3,5-ditert.butyl-2-hydroxybenzyl)-1,2-diamino-ethan-Mangan-Komplex als graugrünen amorphen Feststoff.

Beispiel 6: N,N'-Bis-(2-hydroxybenzoyl)-1,2-diaminoethan-Mangan-Komplex

Zu einer Lösung von 21,6 g N,N'-Bis-(2-hydroxybenzoyl)-1,2-diaminoethan, [hergestellt in Anlehnung an: Y. A. Ibrahim, A.H. M. Elwahy, Synthesis 503-508 (1993)] in 30 ml Ethanol wurden portionsweise 1,86 g Mangen-(II)-acetat (Tetrahydrat) zugegeben und drei Stunden unter Rückfluß zum Sieden erhitzt. Nach Abkühlen der Reaktionslösung wurde der ausgefallene Niederschlag abgetrennt, mit Ethanol gewaschen und getrocknet; man erhielt 2,88 g N,N'-Bis-(2-hydroxybenzoyl)-1,2-diaminoethan-Mangan-Komplex.

Beispiel 7: N,N'-Bis-(2-hydroxybenzoyl)-1,2-diaminoethan-Mangan-Komplex

Der N,N'-Bis-(2-hydroxybenzoyl)-1,2-diaminoethan-Mangan-Komplex wurde wie in Beispiel 6 beschrieben synthetisiert; dabei wurde in der Umsetzung anstelle von Mangan-(II)-acetat (Tetrahydrat) Mangan-(III)-acetat (Dihydrat) eingesetzt.
In analoger Weise wurden die folgenden Metallkomplexe hergestellt.

Beispiel 8: N-(2-Hydroxybenzoyl)-N'-(3,5-ditert.butyl-2-hydroxybenzyliden)-1,2-diaminoethan-Mangan-Komplex

Beispiel 9: N-(2-Hydroxybenzoyl)-N'-(2-hydroxy-3-methoxybenzyliden)-1,2-diaminoethan-Mangan-Komplex

Beispiel 10: N-(2-Hydroxybenzoyl)-N'-(2-hydroxy-4-methoxybenzyliden)-1,2-diaminoethan-Mangan-Komplex

Beispiel 11: N-(2-Hydroxybenzoyl)-N'-(4-diethylamino-2-hydroxybenzyliden)-1,2-diaminoethan-Mangan-Komplex

Beispiel 12: N-(2-Hydroxybenzoyl)-N'-(2-hydroxy-3-methoxybenzyl)-1,2-diaminoethan-Mangan-Komplex

Beispiel 13: N-(2-Hydroxybenzoyl)-N'-(2-hydroxy-4-methoxybenzyl)-1,2-diamino-ethan-Mangan-Komplex

Beispiel 14: N-(2-Hydroxybenzoyl)-N'-(4-diethylamino-2-hydroxybenzyl)-1,2-diaminoethan-Kobalt-Komplex

Bleichversuche

Durch Zusammengeben von 200 ml einer wäßrigen Lösung des Referenzwaschmittels WMP (Wäschereiforschung Krefeld, 5 g/l in Wasser mit 15° dH), 150 mg Natriumperborat-Monohydrat, 50 mg Tetraacetylethylendiamin (TAED) und 2 mg des jeweiligen Katalysators wurde ein Bleichmittel hergestellt. Mit dieser Zusammensetzung wurden mit der Standardanschmutzung BC-1-Tee (Wäschereiforschung Krefeld) verschmutzte Gewebestücke in einem Linitest-Gerät (Heraeus) einer Behandlung bei einer Temperatur von 40 °C unter isothermen Waschbedingungen unterworfen. Nach einer dreißigminütigen Waschzeit wurden die Gewebestücke mit Wasser gespült, getrocknet und gebügelt; anschließend wurde die Bleichwirkung durch eine Bestimmung der Differenzen $\Delta\ R_{(KAT\text{-}TAED)}$ der Remissionen vor und nach dem Bleichen mittels eines Weißgrad-Meßgerätes ELREPHO 2000 (Firma Datacolor) quantifiziert. Aus diesen $\Delta\ R_{(KAT\text{-}TAED)}$-Werten und den in Kontrollversuchen ohne Bleichkatalysator ermittelten $\Delta\ R_{(TAED)}$-Werten wurden die in Tabelle 1 aufgelisteten ΔΔ R-Werte berechnet, die einen direkten Maßstab für die durch den Zusatz an Katalysator hervorgerufene Verbesserung der Bleichwirkung darstellen:

$$\Delta\Delta\ R = \Delta\ R_{(Kat\text{-}TAED)} - \Delta\ R_{(TAED)}$$

Tabelle 1

| Katalysator aus Beispiel-Nr. | 1 | 3 | 5 | 6 | 8 | 9 | 10 | 11 | 12 | 13 | 14 |
|---|---|---|---|---|---|---|---|---|---|---|---|
| ΔΔ R | 2,5 | 4,0 | 3,5 | 3,0 | 2,6 | 2,2 | 2,6 | 4,1 | 4,1 | 4,2 | 3,3 |

Weitere vorteilhafte Eigenschaften der beschriebenen Komplexe sind geringe Farbschädigung und geringe Faserschädigung.

## Patentansprüche

**1.** Verbindungen der allgemeinen Formel 1

$$[L_nM_mX_p]^zY_q \qquad (1)$$

wobei

- M für Mangan in der Oxidationsstufe II, III, IV, V und/oder VI oder Kobalt in der Oxidationsstufe II und/oder III oder Eisen in der Oxidationsstufe II und/oder III,
- X eine Koordinations- oder Brückengruppe,
- Y ein Gegenion in der entsprechenden stöchiometrischen Menge zum Ausgleich einer vorhandenen Ladung z, wobei
- z als Ladung des Metall-Komplexes positiv, null oder negativ sein kann,
- n und m unabhängig voneinander ganze Zahlen von 1 bis 4,
- p eine ganze Zahl von 0 bis 15,
- q z/Ladung von Y
- L einen Liganden der Formel (2), (3) oder (4)

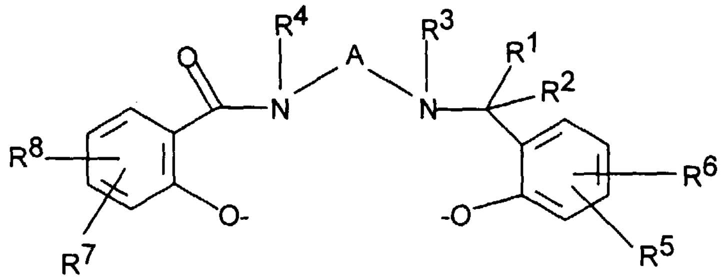

(2)

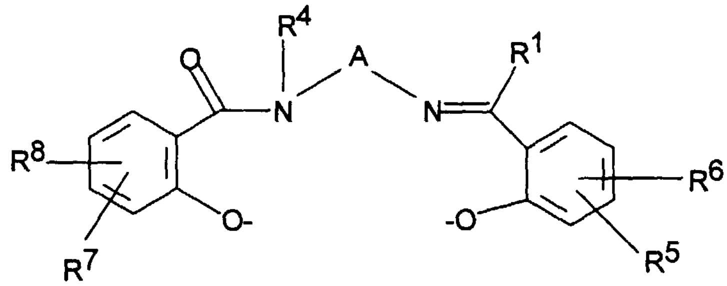

(3)

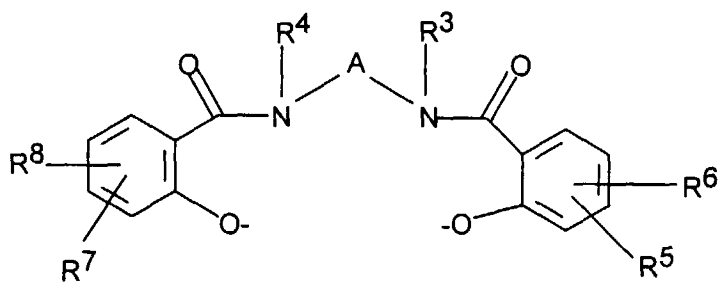

(4)

bedeutet, wobei

| | |
|---|---|
| $R^1$, $R^2$, $R^3$, $R^4$ | unabhängig voneinander Wasserstoff, einen $C_1$- bis $C_{10}$-Alkyl-, Cycloalkyl- oder Arylrest, |
| $R^5$, $R^6$, $R^7$, $R^8$ | unabhängig voneinander Wasserstoff, $C_1$- bis $C_{30}$-Alkyl-, Cycloalkyl- oder Arylreste, $C_1$- bis $C_4$-Alkoxygruppen, substituierte oder unsubstituierte Amino- oder Ammoniumgruppen, Halogenatome, Sulfogruppen, Carboxylgruppen, oder Gruppierungen der Formel $-(CH_2)_r-COOH$, $-(CH_2)_r-SO_3H$, $-(CH_2)_r-PO_3H_2$, $-(CH_2)_l-OH$ bedeuten, wobei r eine ganze Zahl von 0 bis 4 und l eine ganze Zahl von 1 bis 4 bedeutet und die genannten Säuregruppen auch in Salzform vorliegen können und |
| A | für einen $C_2$- bis $C_4$-Alkylenrest, einen $C_5$- bis $C_{10}$-Cycloalkylen- oder einen Arylenrest steht, ausgenommen Verbindungen der Formel 1 wenn L ein Ligand der Formel 4, M = Co, Fe, A = Ethylen, $R^3$ und $R^4$ Wasserstoff und $R^5$ bis $R^8$ Chlor sind. |

**2.** Verbindungen der Formel 1 nach Anspruch 1, wobei X $F^-$, $Cl^-$, $Br^-$, $SCN^-$, $OH^-$, $O_2^{2-}$, $O^{2-}$, $O_2^-$, $HOO^-$, $R^9OO^-$, $H_2O$, $SH^-$, $CN^-$, $OCN^-$, $S^{2-}$, $N_3^-$, $NH_3$, $NR^9_3$, $NR^9_2^-$, $R^9O^-$, $R^9COO^-$, $R^9SO_3^-$ und $R^9SO_4^-$, ist wobei $R^9$ jeweils Wasserstoff, $C_1$- bis $C_8$-Alkyl, Cyloalkyl oder $C_6$- bis $C_{18}$-Arylrest bedeutet.

3. Verbindungen der Formel 1 nach Anspruch 1 wobei das Gegenion Y $F^-$, $Cl^-$, $Br^-$, $NO_3$-, $ClO_4^-$, $SCN^-$, $PF_6^-$, $R^9SO_4^-$, $R^9COO^-$, $R^9SO_3^-$, $BF_4^-$, $BPh_4^-$, $SO_4^{2-}$ und $SO_4^{2-}$; $Li^+$, $Na^+$, $K^+$, $Mg^{2+}$, $Ca^{2+}$, $Al^{3+}$, $NH_4^+$, $R^9NH_3^+$, $R^9{}_2NH_2^+$, $R^9{}_3NH^+$ und $R^9{}_4N^+$ bedeutet und $R^9$ die in Anspruch 2 genannte Bedeutung hat.

4. Verbindungen der Formel 1 nach einem oder mehreren der Ansprüche 1 bis 3, wobei M Mangan in der Oxidationsstufe II, III, IV, V und/oder VI bedeutet, m und n jeweils für die Zahlen 1 oder 2 stehen und p eine ganze Zahl von 0 bis 5 bezeichnet.

5. Verbindungen der Formel 1 nach einem oder mehreren der Ansprüche 1 bis 3, wobei M Mangan in der Oxidationsstufe II und/oder III bedeutet, m und n jeweils für die Zahlen 1 oder 2 stehen und p eine ganze Zahl von 0 bis 5 bezeichnet.

6. Verbindungen der Formel 1 nach einem oder mehreren der Ansprüche 1 bis 3, wobei M Mangan in der Oxidationsstufe II, III, IV, V und/oder VI bedeutet, n und m für die Zahl 1 und p für eine ganze Zahl von 0 bis 3 steht.

7. Verbindungen der Formel 1 nach einem oder mehreren der Ansprüche 1 bis 3, wobei M Mangan in der Oxidationsstufe II und/oder III bedeutet, n und m für die Zahl 1 und p für eine ganze Zahl von 0 bis 3 steht.

8. Verbindungen der Formel 1 nach einem oder mehreren der Ansprüche 1 bis 3, wobei M Mangan in der Oxidationsstufe II, III, IV, V und/oder VI bedeutet, n und m für die Zahl 1 steht und p Null bedeutet.

9. Verwendung der Verbindungen der Formel 1 gemäß Anspruch 1 als Blech- und Oxidationskatalysatoren.

10. Verwendung der Verbindungen der Formel 1 gemäß Anspruch 1 als Bleich- und Oxidationskatalysatoren in Wasch- und Reinigungsmitteln und bei der Textil- und Papierbleiche.